# EUROPEAN PATENT APPLICATION

(11) **EP 2 292 277 A2**
(43) Date of publication of application: **09.03.2011**
(21) Application number: 10251343.9
(22) Date of filing: 28.07.2010
(51) Int. Cl.: A61L 17/14

(54) **Polymer coated sutures**

(30) Priority: 29.07.2009 US 511187
(71) Applicant: Tyco Healthcare Group LP, North Haven, CT 06473 (US)
(72) Inventor: Abuzaina, Ferass, Shelton, CT 06484 (US); Irfan, Ali, Shelton, CT 06484 (US); Stopek, Joshua A., Guilford, CT 06437 (US); Cuevas, Brian, Cumming, GA 30040 (US); Hotter, Joseph, Middletown, CT 06457 (US)
(74) Representative: Soames, Candida Jane

(57) **Abstract**

The present disclosure describes a coating for a surgical suture including a vinyl lactam polymer and a lactone polymer.

## Description

### CROSS-REFERENCE TO RELATED APPLICATION

This application is a continuation in part of U.S. Patent Application Ser. No. 11/789,531, filed on April 25, 2007, the entire disclosure of which is incorporated herein by reference.

### BACKGROUND

### Technical Field

The present disclosure relates to surgical sutures, and more particularly to coated surgical sutures displaying enhanced knot security.

### Background of Related Art

Surgeons are constantly seeking better and stronger knot-tying materials and methods. This is true in the fields of arthroscopy and laparoscopy, as well as in the field of open procedures. Arthroscopic and laparoscopic procedures, however, may be more technically demanding due to limited accessibility, as compared to open procedures. For example, the task of tying secure knots may prove to be more difficult during an arthroscopic procedure considering that the surgeon is required to tie the suture knot away from the defect and use a knot pusher to slide and/or tension the knot into position. Of course, whether performed arthroscopically, laparoscopically, or openly, suture knots must be securely tied and provide optimal knot security (resistance to loosening and/or slipping of the knot).

In arthroscopic procedures such as meniscal repair, the suture knots commonly consist of an initial sliding knot which is followed by a series of half-hitches to prevent slack in the slip knot. The addition of the half-hitches enhances knot security but also produces a larger knot profile. Knots having a larger profile may rub against the surrounding tissue causing pain and discomfort. In more severe situations, the larger knots may rub against the cartilage resulting in the formation of osteoarthritis. Also, larger knots place larger amounts of suture material into the body thereby increasing the likelihood of developing inflammation and/or infection at or near the site of the knot. Therefore, it would be beneficial to provide a suture knot which displays enhanced knot security without the need to increase the knot profile.

### SUMMARY

The present disclosure describes coated sutures that display an enhanced knot security. The suture knots also form smaller profile knots when tied and cinched. The sutures are coated with a composition which includes a blend of a vinyl lactam polymer and a lactone copolymer. In some embodiments, the coating may further include an ester of a fatty acid, such as calcium stearoyl lactylate. In some embodiments, the coating may also include hyaluronic acid.

Methods are also disclosed wherein a suture made from a biocompatible material is provided. The suture is coated with a blend of a polymers derived from a vinyl lactam polymer and a lactone polymer. The coated suture is dried and then tied into a preformed, uncinched knot. In embodiments, the knot is a sliding knot.

### BRIEF DESCRIPTION OF THE DRAWINGS

Various embodiments of the present disclosure will be discussed in more detail below in conjunction with selected embodiments and the appended drawings wherein:

FIG.1 shows a perspective view of one embodiment of a coated suture according to the present disclosure; and

FIGS. 2A-2F show a perspective view of other embodiments of a coated suture according to the present disclosure;

### DETAILED DESCRIPTION

The present disclosure describes surgical sutures which display enhanced knot-security when tied into a knot and cinched. The sutures are coated with a suture coating. The suture coating includes a blend of at least one polymer derived from a vinyl lactam polymer and at least one polymer derived from a lactone polymer. In some embodiments, the suture coating may further include an ester of a fatty acid, such as calcium stearoyl lactylate. In some embodiments, the suture coating may also include hyaluronic acid. The coated sutures may be monofilaments or multifilament devices.

The sutures can be formed from any sterilizable biocompatible material that has suitable physical properties for the intended use of the suture. The sutures can be made from synthetic or natural polymers that are either bioabsorbable or non-bioabsorbable. Some specific non-limiting examples of suitable absorbable materials include trimethylene carbonate, caprolactone, dioxanone, glycolic acid, lactic acid, glycolide, lactide, homopolymers thereof, copolymers thereof, and combinations thereof. Some specific non-limiting examples of suitable non-absorbable materials which may be utilized to form the sutures include polyolefins, such as polyethylene, polypropylene, copolymers of polyethylene and polypropylene, and blends of polyethylene and polypropylene. In embodiments, the suture may be made from a polyester material, such as polyethylene terephthalate. In some embodiments, the suture may be made from an ultra-high molecular weight polyethylene. It should, also be understood that combinations of materials may be used to form the sutures.

Methods for preparing materials suitable for making sutures as well as techniques for making sutures from such materials are within the purview of those skilled in the art.

The sutures described herein are coated with a suture coating which contains a blend of polymers derived from a vinyl lactam polymer and lactone polymer. Any vinyl lactam polymer may be included in the blend used to form the coating. As used herein, the term "vinyl lactam polymer" means any polymer that may be derived from, synthesized from, or prepared with at least one lactam monomer of the following formulas: 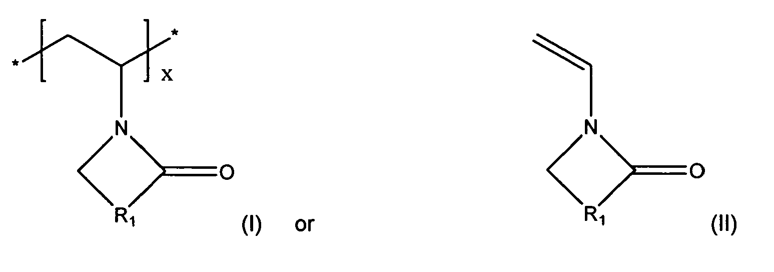 wherein R₁ represents a C₁-C₄ alkyl, alkenyl, or alkadienyl bridge group necessary to complete a 4, 5, 6 or 7-membered heterocyclic ring system; and x represents an integer from 0 to 50.

In some embodiments, the vinyl lactam polymer includes at least one of the following monomeric units: N-vinyl-2-pyrrolidone, N-vinyl-2-piperidone, and N-vinyl-caprolactam, N-vinyl methyl pyrrolidone, N-vinyl ethyl pyrrolidone, N-vinyl methyl caprolactam, vinyl pyrrolidone, vinyl caprolactam, vinyl piperidone and combinations thereof. The vinyl lactam polymer may be a homopolymer or a copolymer, such as a random copolymer, a block copolymer, graft copolymer or a segmented copolymer. Any monomer which can be copolymerized with a vinyl lactam monomer to provide a biocompatible polymer may be used to form the copolymer. Suitable comonomers includes, but are not limited to, hydrophilic vinyl monomers, such as methacrylic acid, acrylic acid, acrylamide, sodium acrylate, sulfopropyl acrylate, sulfopropyl methacrylate, vinyl functionalized phosphorylcholine, hydroxyl ethyl methacrylate, methacrylamide, niipam and the like. In embodiments, the vinyl lactam polymer is polyvinyl pyrrolidone (PVP).

Generally, the vinyl lactam polymer may represent up to about 75% by weight of the blend. In some embodiments, the vinyl lactam polymer may represent from about 20% to about 67 % by weight of the blend.

The vinyl lactam polymer may be combined with a lactone polymer to form the blend used in coating the suture. Any known lactone polymer may be included in the blend used to form the suture coating. As used herein, the term "lactone polymer" means any polymer that may be derived from, synthesized from, or prepared with at least one lactone monomer of the following formula: 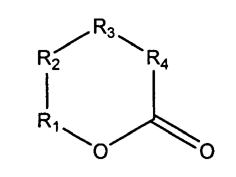 wherein the R₁. R₂, R₃ and R₄ groups may independently represent one of the following, an oxygen atom, a carbonyl group, or a linear, branched or cyclic C₁ to C₂₀ alkyl, alkenyl, alkadienyl group.

The lactone polymer may be a homopolymer or a copolymer, such as a random copolymer, a block copolymer, graft copolymer, or a segmented copolymer. Any monomer which can be copolymerized with a lactone monomer to provide a biocompatible polymer may be used to form the copolymer. Suitable comonomers include, but are not limited to alkylene oxides, acrylates, acrylamides and saccharides (e.g., dextrin). In embodiments the lactone polymer includes at least one of the following monomeric units: lactide, glycolide, caprolactone, dioxanone, propiolactone, butyrolactone, valerolactone, decalactone, pivalolactone, stearolactone, palmitolactone, trimethylene carbonate and combinations thereof. In some embodiments, the lactone polymer contains caprolacatone, glycolide, lactide and combinations thereof.

Generally, the lactone polymer may represent up to about 85% by weight of the blend. In some embodiments, the lactone polymer may represent from about 40% to about 80 % by weight of the blend. In some embodiments, the lactone may represent more than 50% by weight of the blend.

The vinyl lactam polymer and the lactone polymer may be combined in any solvent suitable for use in making surgical sutures. In some embodiments, the blend including a vinyl lactam polymer and a lactone polymer may be combined in a solvent which both polymers are mutually soluble to form the suture coating. Examples of suitable solvents include but are not limited to chlorinated solvents, chloroform, methylene chloride (MC), methyl ethyl ketone (MEK), 1,1,2-trichloroethane, trifluoroethanol, methyl acetate, ethyl acetate, amyl acetate, acetone, tetrahydrofuran, 1,3-dioxolane, 1,4-dioxane, cyclohexanone, ethyl formate, 2,2,2-trifluoroethanol, 2,2,3,3-tetrafluoro-1-propanol, 1,3-difluoro-2-propanol, 1,1,1,3,3,3-hexafluoro-2-methyl-2-propanol, 1,1,1,3,3,3-hexafluoro-2-propanol, 2,2,3,3,3-pentafluoro-1-propanol, and nitroethane.

The blend of polymers derived from a vinyl lactam polymer and lactone polymer may represent up to about 10% by weight of the suture coating. In some embodiments, the blend of polymers derived from a vinyl lactam polymer and lactone polymer may represent from about 0.1 % to about 7.5 % by weight of the suture coating. In some embodiments, the blend of polymers derived from a vinyl lactam polymer and lactone polymer may represent from about 3% to about 5% by weight of the suture coating.

The vinyl lactam polymer and lactone polymer may also be combined with an ester of a fatty acid to form the suture coating. Some non-limiting examples of suitable fatty acids include: calcium, magnesium, aluminum, barium, or zinc stearoyl lactylate; calcium, magnesium, aluminum, barium, or zinc palmityl lactylate; and, calcium, magnesium, aluminum, barium, or zinc olelyl lactylate. In embodiments, the suture coating may include a blend of a vinyl lactam polymer, a lactone polymer and a calcium stearoyl lactylate.

The fatty acid ester may represent up to about 33% by weight of the blend. In embodiments, the fatty acid ester may represent from 0.1% to about 20% by weight of the blend.

In some embodiments, the suture coating may also include hyaluronic acid. Hyaluronic acid is a non-sulfated glycosaminoglycan with a molecular weight ranging from about 35,000 to about 350,000. In some embodiments, the coating includes hyaluronic acid with a molecular weight of about 72,000. The hyaluronic acid may represent from about 0.01% to about 10% by weight of the suture coating. In some embodiments, the hyaluronic acid may represent from about 0.05% to about 5% by weight of the suture coating. In some other embodiments, the hyaluronic acid may represent about 0.1 % to about 1% by weight of the suture coating.

In still other embodiments, the suture coatings may further include optional ingredients. Optional ingredients, as used herein, may represent up to about 20% by weight of the suture coating. Some optional ingredients include but are not be limited to dyes, viscosity-enhancers, emulsifiers, surfactants, fragrances, contrast agents (such as Barium and Barium containing polymers), as well as drugs such as antimicrobials, antivirals, chemotherapeutics, antiseptics, analgesics, anti-inflammatory agents, polymeric-drugs, combination thereof, and the like. Some other examples include proteins, growth factors, hormones, genetic materials, cellular materials and the like. The optional ingredients may be mixed with the biocompatible suture material prior to the formation of the suture. In some embodiments, the optional ingredients may be added to the suture coating. In still other embodiments, the optional ingredients may be added to the suture through a chemical or physical deposition or impregnating process.

The coatings described herein may be applied to a suture by any technique, including but not limited to dipping, spraying, brushing, wiping, or any other appropriate technique for forming a continuous layer onto the surface of an implantable device. The particular technique used may be chosen by those skilled in the art based on a variety of factors such as the specific construction of the surgical suture and the material contained in the coating.

The coated sutures may be dried using any suitable technique, including but not limited to, the use of an oven. In some embodiments, the coated suture may be dried under vacuum at a temperature of about 40° C. In some embodiments, a tunnel oven may be used to drive off the solvent of the coating on the suture.

After drying, the coated sutures may be tied into preformed, uncinched knots. A pre-formed, uncinched knot is a suture knot that is loosely tied. A preformed, uncinched knot needs to be pulled taut to tighten and/or seat the knot. The profile of a pre-formed, uncinched knot may be decreased after being pulled taut. The preformed, uncinched knots may be configured manually or by machine, depending upon the complexity of the configuration of the knot.

The coated pre-tied sutures may be used in any type of surgical procedure. However, because the coated pre-tied sutures may display a tensile strength of greater than about 40N the coated pre-tied sutures may be particularly useful during arthroscopic procedures including meniscal repair surgeries.

Unlike the sutures of the prior art, the coated sutures described herein do not require the addition of half-hitches to display an enhanced knot security. Rather, the coated sutures described herein maintain the smallest knot profile while displaying an enhanced knot security equal to or greater than other larger profile knots which include multiple half-hitches. In addition, because the pre-tied coated sutures of the present disclosure produce smaller knot profile, the pre-tied knots also position less suture material at the sight of implantation which decreases the likelihood of initiating an immune response to the suture during degradation.

In embodiments, the preformed, uncinched knot may be a sliding knot. Some examples of sliding knots include but are not meant to be limited to, the Duncan loop, the Tautline (or Midshipman) hitch, the Tennessee slider, the Roeder knot, the Weston knot, the Hangman's loop, the SMC (Samsung Medical Center) knot, the Modified SMC knot, the Giant knot, the Nicky's knot, the Double-Twist knot, the Lafosse knot and the Easy knot. In some embodiments, the coated suture may be used to form an SMC knot, which may be used in arthroscopic shoulder surgery.

The preformed knots may be cinched into position by any suitable technique. For instance, the preformed knot may simply be cinched into position by hand. In some embodiments, a surgeon may use the assistance of a surgical device known as a knot pusher. Knot pushers move the preformed knot down a length of the suture without deforming or collapsing the knot. Some knot-pushing devices require further manual interaction by the surgeon, while other knot-pushing devices are machine operated. Any suitable knot-pusher may be used with the preformed, uncinched knots described herein.

In some embodiments, a wetting agent may be applied to the dried sutures prior to the tying of the knot. In some embodiments, a wetting agent may be applied to the dried suture prior to the cinching of the preformed knot. The wetting agent temporarily improves the ability of the dried suture to slide down a length of the suture during the knotting process. Some examples of suitable wetting agents include, but are not limited to, water, saline solution, dextrose solution, and the like. The wetting agent may applied to the coated suture using any suitable technique including dipping.

The coated sutures also may be used in combination with other fixation devices. For instance the may be tied into a preformed, uncinched knot which may be attached to a suture anchor. Some other non-limiting examples of suitable fixation devices include screws, pins, clips, bone plates and the like.

In some embodiments, the coated sutures described herein may be tied into preformed, uncinched knots for use in arthroscopic or laparoscopic procedures. Although suitable for use in any procedures, the preformed, uncinched knots may be useful for repair of a torn meniscus. Meniscal repair may require a knotted suture to withstand pull-out forces greater than about 20N. In embodiments, the coated sutures described herein may form knots which remain secure to pull-out forces greater than about 40N. In some embodiments, the coated sutures described herein may form knots which remain secure to pull out forces ranging from about 40N to about 75N.

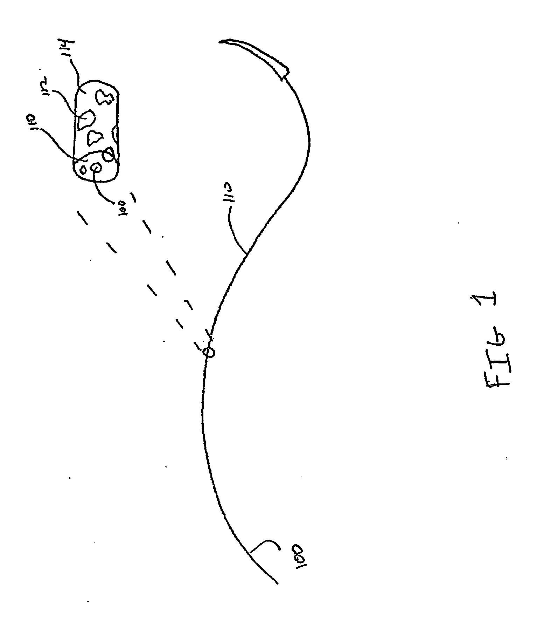

Turning now to FIG. 1, suture 100 is shown having a coating 110 and a needle 120. Suture 100 is made from any biocompatible material and coating 110 includes a blend of polymers derived from a vinyl lactam polymer and a lactone polymer. Although coating 110 is shown to cover the entire length of suture 100, it is envisioned that the coating may only cover a portion of the suture. In some embodiments, the coating may phase-separate after drying wherein vinyl lactam polymer and lactone polymer are positioned on different portions of the suture.

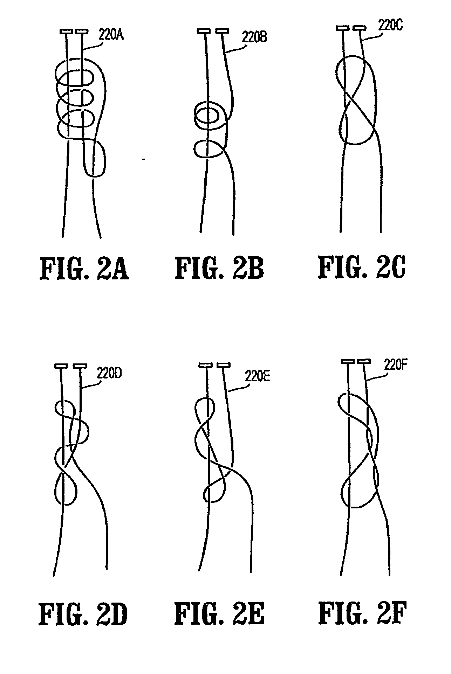

In FIGS. 2A-2F, coated sutures 220A-F are shown in a variety of preformed, uncinched knots. More specifically, coated sutures 220A-F are shown in the following preformed, uncinched knot configurations: Duncan's Loop, Nicky's Knot, Tennessee Slider, Roeder Knot, SMC knot and Weston Knot, respectively.

The coated sutures may be sterilized and supplied in sealed medical device packages. The sutures maybe sterilized using any suitable technique, including exposure the ethylene oxide, and gamma radiation. In some embodiments, the coated sutures may be sterilized and/or packaged after the tying of the preformed, uncinched knot. In some embodiments, the coated sutures may be sterilized and/or packaged prior to the forming of the knot.

### Example 1

Uncoated TICRON^{™ p}olyester sutures were coated with one of the coating formulations listed in Table-1 below. The coating formulation was applied to the TICRON^{™} polyester sutures using either a hand-dipping process or production coating equipment.

The hand-dipped sutures were cut into about 30-inch pieces and dipped into a solution containing one the various coating formulations for 30 seconds. The sutures were then removed from the solution and dried under vacuum at 40° C for 5 hours.

The production coated sutures were coated using equipment supplied by Dietz and Shell. The uncoated TICRON^{™} polyester sutures were passed continuously through a vertically oriented capillary which was continuously flooded with a coating solution. The coated suture was then passed through a tunnel oven to drive off solvent before being take-up on a spool.

Both the hand-dipped and production coated sutures were then tested to evaluate the coating formulations' effect on knot security, using a simple unmodified SMC knot. The knot security of the tied sutures was tested using the following equipment:

| | |
|---|---|
| Instron Model: 4301 (2) | Gauge #SUZ-0667 |
| Load Cell-1OON | Serial # 21451 |
| BlueHill test method: | 52 - Knot Security Determination |
| Test Type -Tension | Test Speed- 51 mm/min |
| Data rate- 50.00 ms | |

The following steps were taken to test the sutures:
1. The base of the Instron was positioned directly below the load cell and load cell was balanced.
2. One end of the split circular loop fixture was attached to the load cell by using a coupling.
3. The other end of the fixture was attached to a vice.
4. The fixtures were safely secured and aligned before the gauge length for the testing was measured.
5. Each suture sample from each specimen was soaked for 2 min in the saline solution.
6. Each suture sample from each specimen was tied into a SMC knot (without any additional throws) and tensioned by pulling the loop limb while pushing on the post with a hot pusher against a 1-inch stainless steel tube.
7. The tied knot was removed and the long ears of the suture were cut to a length of about 3mm.
8. The knot was then soaked in saline for 2 minutes.
9. The knot was then aligned centrally with the two ends of the fixture.
10. Tension was then applied to the knot as described above.
11. The tension was stopped after the knot either slipped or broke.
12. The gauge length was reset after the tension was stopped.
13. Steps 4-12 were repeated for each of the samples.
14. All the coating formulations were run n=10.
15. Coated and uncoated TICRON^{™} polyester controls were run with every batch of samples tested.

Table-1 displays the results of each of the sample coating formulations results. Particularly useful coating formulations result in less than 10% knot slips and average a tensile strength greater than or equal to about 45N (about 5 KgF).

**Table-1**

| **No.** | **Composition** | **Coating Method** | **Coating Condition** | **Tensile Strength (KgF)** | **StD** | **Break %** |
|---|---|---|---|---|---|---|
| 1 | Coated | ** | ** | 0.20 | 0.09 | 0 |
| 2 | Uncoated | N/A | N/A | 3.75 | 1.04 | 16 |
| 3 | Coated** with two ½ hitches | ** | ** | 4.64 | 1.67 | 25 |
| 4 | Uncoated with two ½ hitches | N/A | N/A | 6.22 | 0.61 | 100 |
| 5 | Ethibond Excell*** | N/A | N/A | 2.81 | 0.62 | 0 |
| 6 | 0.1% w/w HA 1.2M in MC | HD | N/A | 4.17 | 1.34 | 20 |
| 7 | 0.35% w/w HA 1.2M in MC | HD | N/A | 3.63 | 0.94 | 10 |
| 8 | 2.5% w/w PBA in MEK | HD | N/A | 3.95 | 0.53 | 10 |
| 9 | 2.67% w/w PBA + 0.27% HA in MEK | HD | N/A | 2.27 | 0.81 | 0 |
| 10 | 2% w/w (80/20 lactide/glycolide) in MC | HD | N/A | 4.95 | 0.49 | 70 |
| 11 | 5% w/w PVP (10K) in MC | HD | N/A | 4.85 | 0.76 | 50 |
| 12 | 5% w/w PVP (55K) in MC | HD | N/A | 5.09 | 0.93 | 70 |
| 13 | 2% w/w PVP (360K) in MC | HD | N/A | 5.44 | 0.51 | 80 |
| 14 | 1% w/w PVP (1.3 Mil) in MC | HD | N/A | 5.16 | 1.14 | 40 |
| 15 | 5% w/w (80/20 lactide/glycolide) in MC | HD | N/A | 4.28 | 1.27 | 50 |
| 16 | 5% w/w (70/30 lactide/glycolide) in MC | HD | N/A | 4.91 | 0.95 | 70 |
| 17 | 5% w/w (70/30 lactide/glycolide) in MC | D&S | 40/100* | 5.44 | 0.54 | 80 |
| 18 | 2.5% w/w (70/30 lactide/glycolide) in MC | D&S | 40/100* | 5.16 | 0.59 | 70 |
| 19 | 5% w/w (70/30 lactide/glycolide) in MC | D&S | 40/60* | 5.56 | 0.65 | 100 |
| 20 | 3.5% (72%(70/30 lactide/glycolide)+28%PVP) | HD | N/A | 5.99 | 0/67 | 100 |
| 21 | 4.5%(72%(70/30 lactide/glycolide)+28%PVP) | HD | N/A | 5.21 | 1.83 | 100 |
| 22 | 3.5%(50%(70/30 lactide/glycolide)+50%PVP) | HD | N/A | 4.97 | 1.51 | 100 |
| 23 | [3.5%(72%(70/30 lactide/glycolide) +28%PVP)] + 01% HA 72K | HD | N/A | 5.49 | 1.11 | 90 |
| 24 | [4.5%(72%(70/30 lactide/glycolide) +28PVP)] + 0.1 % HA 72K | HD | N/A | 5.90 | 0.54 | 90 |
| 25 | [3.5%(50%(70/30 lactide/glycolide) +50PVP)] + 0.1 % HA | HD | N/A | 4.59 | 1.39 | 80 |
| 26 | 5%(50%(70/30 lactide/glycolide) +50% CSL) | HD | N/A | 4.27 | 1.11 | 40 |
| 27 | 5%(80%(70/30 lactide/glycolide) +20% CSL) | HD | N/A | 4.36 | 0.64 | 50 |
| 28 | 2.5%(72% (70/30 lactide/glycolide) + 28% CSL) | HD | N/A | 4.09 | 0.96 | 20 |
| 29 | 3%(67% PVP + 33% CSL) | HD | N/A | 5.13 | 0.87 | 90 |
| 30 | 5% (50%(70/30 lactide/glycolide) + 30% PVP +20% CSL) | HD | N/A | 5.25 | 1.14 | 90 |

| | | | | | | |
|---|---|---|---|---|---|---|
| * Machine Speed/Pump Speed ** TICRON^{™} polyester sutures already coated with silicone ***Ethibond^{™} sutures already coated with PBA HA represents hyaluronic acid MC represents methylene chloride PVP represents polyvinyl pyrrolidone CSL represents calcium stearoyl lactylate PBA represents polybutylate acid | | | | | | |

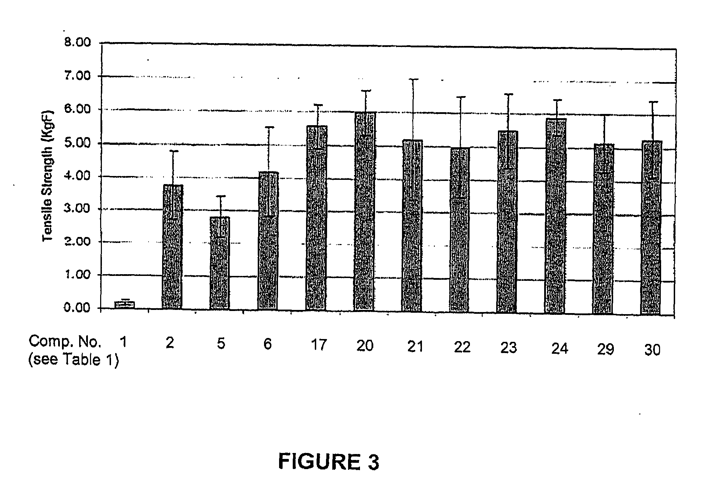

Figure 3 displays the load to failure of the SMC knots coated with each of the different formulations.

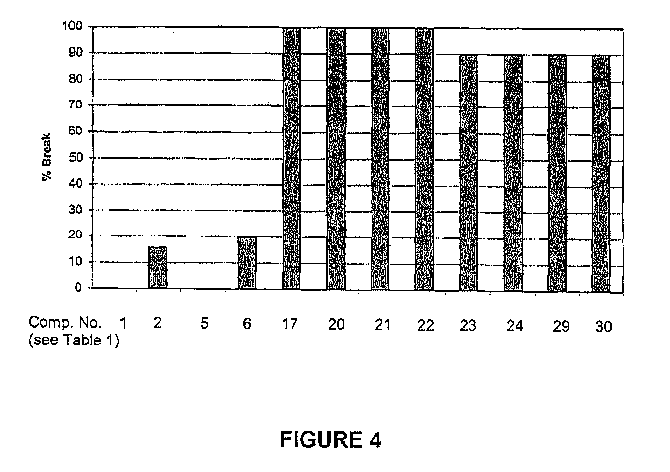

Figure 4 displays the percent of the SMC knots that broke (did not slip) during the tensile test of each of the different formulations.

It will be understood that various modifications may be made to the embodiments disclosed herein. Therefore, the above description should not be construed as limiting, but merely as an exemplification of embodiments. Those skilled in the art will envision other modifications within the scope and spirit of this disclosure. Various modifications and variations of the coated sutures and uses thereof will be apparent to those skilled in the art from the foregoing detailed description. Such modifications and variations are intended to come within the scope of the following claims.

## Claims

1. A suture made of a biocompatible material comprising a suture coating containing hyaluronic acid and a blend of polymers derived from a vinyl lactam polymer and a lactone polymer.

2. The suture of claim 1 wherein the vinyl lactam polymer comprises monomeric units selected from the group consisting of N-vinyl-pyrrolidone, N-vinyl-2-piperidone, and N-vinyl-caprolactam, N-vinyl methyl pyrrolidone, N-vinyl ethyl pyrrolidone, N-vinyl methyl caprolactam, and combinations thereof, or the suture of claim 1 wherein the vinyl lactam polymer comprises polyvinyl pyrrolidone.

3. The suture of claim 1 or claim 2 wherein the vinyl lactam polymer represent up to about 75% by weight of the blend, preferably wherein the vinyl lactam polymer represents from about 20% to about 67% by weight of the blend.

4. The suture of any preceding claim wherein the lactone polymer comprises polymers derived from monomeric units selected from the group consisting of lactide, glycolide, caprolactone, dioxanone, propiolactone, butyrolactone, valerolactone, decalactone, pivalolactone, stearolactone, palmitolactone, trimethylene carbonate and combinations thereof.

5. The suture of claim 4 wherein the lactone polymer represents up to about 85% by weight of the blend, preferably wherein
the lactone polymer represents from about 40% to about 80% by weight of the blend.

6. The suture of any preceding claim wherein the hyaluronic acid represents from about 0.01% to about 10% by weight of the coating; preferably wherein the hyaluronic acid represents from about 0.05% to about 10% by weight of the coating; preferably still wherein the hyaluronic acid represents about 0.01 % by weight of the coating.

7. The suture of any preceding claim wherein the hyaluronic acid comprises a molecular weight ranging from about 35,000 to about 350,000, preferably wherein the hyaluronic acid comprises a molecular weight of about 72,000.

8. The suture of any preceding claim wherein the blend represents up to about 10% of the suture coating; preferably wherein the blend represents from about 0.1% to about 7.5% by weight of the suture coating, preferably still wherein the blend represents from about 3% to about 5% by weight of the suture coating.

9. A method comprising:
providing a suture made of a biocompatible material;
coating the suture with a blend of polymers derived from a vinyl lactam polymer and a lactone polymer;
drying the coated suture; and
tying the coated suture into an preformed, uncinched knot.

10. The method of claim 9 wherein the suture comprises a biocompatible material selected from the group consisting of trimethylene carbonate, caprolactone, dioxanone, glycolic acid, lactic acid, polyethylene, polypropylene and combinations thereof.

11. The method of claim 10 wherein the suture comprises polyethylene terephthalate; and/or the method of claim 10 wherein the suture comprises an ultra-high molecular weight polyethylene.

12. The method of any of claims 9 to 11, wherein the vinyl lactam polymer comprises monomeric units selected from the group consisting of N-vinyl-pyrrolidone, N-vinyl-2-piperidone, and N-vinyl-caprolactam, N-vinyl methyl pyrrolidone, N-vinyl ethyl pyrrolidone, N-vinyl methyl caprolactam, and combinations thereof or the method of any of claims 9 to 11, wherein the vinyl lactam polymer comprises polyvinyl pyrrolidone.

13. The method of any of claims 9 to 12 wherein the lactone polymer comprises polymers derived from monomeric units selected from the group consisting of lactide, glycolide, caprolactone, dioxanone, propiolactone, butyrolactone, valerolactone, decalactone, pivalolactone, stearolactone, palmitolactone, trimethylene carbonate and combinations thereof.

14. The method of any of claims 9 to 13 wherein the coating further comprises hyaluronic acid, or the method of any of claims 9 to 13 wherein the coating further comprises calcium stearoyl lactylate.

15. The method of any of claims 9 to 14 wherein drying of the coated suture is performed under vacuum at about 40° C.

16. The method of any of claims 9 to 14 wherein the suture is tied into a slidable knot, wherein the slidable knot is selected from the group consisting of the Duncan loop, the Tautline hitch, the Tennessee slider, the Roeder knot, the Weston knot, the Hangman's loop, the SMC knot, the Giant knot, the Nicky's knot, the Double-Twist knot, the Lafosse knot and the Easy knot; preferably wherein the suture is tied into a SMC knot.

17. The method of claim 16 further comprising the step of applying a wetting agent to the dried suture prior to cinching the preformed knot, preferably wherein the wetting agent is saline solution.
